# EUROPEAN PATENT APPLICATION

(11) **EP 0 712 929 A2**
(43) Date of publication of application: **22.05.1996**
(21) Application number: 95118319.3
(22) Date of filing: 21.11.1995
(51) Int. Cl.: C12N 15/00, A01K 67/027, C07K 14/47, C12N 15/12

(54) **A transgenic nonhuman animal deficient in T-cells**

(30) Priority: 21.11.1994 KR 9430675
(71) Applicant: Seo, Jeongsun, Kang-nam-ku, Seoul (KR)
(72) Inventor: Seo, Jeongsun, Kangnam-ku, Seoul (KR); Kim, Soonhee, Songpa-ku, Seoul (KR); Park, Woongyang, Chongno-ku, Seoul (KR)
(74) Representative: VOSSIUS & PARTNER

(57) **Abstract**

The present invention relates to a transgenic nonhuman animal deficient in T-cells, which is provided by fusing heat shock protein (Hsp) gene with a promoter of a gene expressed in thymus and transferring it to an animal. A transgenic nonhuman animal with a shrunken thymus and a deficiency in mature T-cells can be obtained. This invention is useful for the fields of research of autoimmune disease genesis mechanisms, immune inhibitors and novel drugs.

## Description

The present invention relates to a recombinant DNA comprising a heat shock protein 70 gene operatively linked downstream to a promoter of a gene expressed in thymus, a transgenic nonhuman animal deficient in T-cells, and a process for preparing the transgenic nonhuman animal.

Transgenic mice can be created by microinjecting a particular gene fragment into a fertilized egg of a mouse, introducing it into the womb of another mouse, which will give birth to pups, screening the tail DNA of the offspring 3 weeks after birth, and confirming that the gene is inserted into DNA in the cells of the offspring. A transgenic mouse is useful for identifying functions of a gene. Improvement of models for many human diseases, medicines against the diseases, and bio-reactors converting the body of a mouse to a biofactory are expected fields of applicability of a transgenic mouse.

Recently, a novel model according to the transgenic mouse technique as an approach to genetic problems such as cancer has been developed. The success of a c-myc oncogene-inserted transgenic mouse by P. Leder at Harvard University in the U.S.A. has drawn great attention in the field of human leukemia and this technique has become the subject of a patent (U.S. Pat. No. 5,087,571). U.S. Pat. No. 5,175,383, issued to Philip Leder et al., describes male mice with giant prostate glands, U.S. Pat No. 5,175,384 issued to Paulus J.A. Krimpenfort et al., describes mice with an inability to produce mature T-cells, and U.S. Pat. No. 5,175,385, issued to Thomas E. Wagner et al., introduces mice which produce human β-interferon.

The immune response is a complex defense system which is able to recognize and kill invading organisms such as bacteria or viruses, or unusual cells produced in self-tissues. The most characteristic aspects of the immune system are the specific recognition of antigens and the ability to discriminate between self- and non-self antigen. It consists of a humoral immune system comprised of B-cells and a cellular immune system comprised of T-cells. The lymphocytes of the peripheral blood consist of 65-80% T-cells and 8-15% B-cells. T-cells outnumber B-cells and, moreover, function in the total regulation of the immune system, which regulates antibody production and prevents-injury to target cells. The hematogenous liver cells enter the bone marrow at an embryonic stage and then pass through the thymus to become T-cells. Accordingly, the thymus is a necessary organ for the formation of mature T-cells.

Heat shock protein (Hsp) is an important protein in repairing damage to cells, is expressed in the state of heat shock or stress, at an embryogenic stage as well as during a special step of cell differentiation and the cell cycle (Hunt, C. and Morimoto, R.I., 1985, Proc. Natl. Acad. Sci. USA, 82 (19), 6455-6459). Also, the H2K promoter, which is a promoter of an H-2 histocompatability gene, is activated in various cell types of spleen, pancreas, thymus, lung, etc. (Rüther et al., 1988, Cell, Vol. 53, 847-856).

This invention arose from research on the influence of expression of Hsp on many organs and cell types and the observation that a transgenic mouse containing a recombinant Hsp gene and an H2K promoter has a considerably shrunken thymus and is deficient in mature T-cells.

Accordingly, it is an object of this invention to provide a transgenic nonhuman animal deficient in T-cells by transferring a gene, which is produced by recombining an Hsp gene with a promoter of a gene expressed in thymus, such as an H2K promoter, to a host animal.

The present invention provides a recombinant DNA, comprising a promoter of a gene expressed in thymus and a heat shock protein 70 gene operatively linked downstream to said promoter, and a transgenic nonhuman animal comprising said recombinant DNA.

Further, this invention provides a process for preparing a transgenic nonhuman animal comprising the steps of inserting the recombinant DNA comprising the promoter of a gene expressed in thymus and heat shock protein 70 gene operatively linked downstream to said promoter into a fertilized egg, transferring said fertilized egg to a foster mother, and obtaining the offspring of said mother. The invention also includes a transgenic nonhuman animal prepared by the above process.

The preferred promoter of a gene expressed in thymus is an H2K gene promoter. Further preferred is a human H2K gene promoter. The preferred heat shock protein 70 gene is a human heat shock protein 70 gene.

The promoter of said H2K gene may be prepared by digesting H2K gene with EcoRI and HindIII restriction enzymes. Said heat shock protein 70 gene may be prepared by digesting a heat shock protein 70 gene with BamHI and HindIII restriction enzymes. The HindIII site of said H2K promoter can then be ligated to the HindIII site of said heat shock protein 70 gene. Other promoters of genes expressed in thymus may be similarly recombined with Hsp 70 gene using methods well known in the art.

The host animal may be selected, for example, from the group consisting of mouse, pig and chicken. In a preferred embodiment, the host animal is a mouse.

The recombinant DNA is preferably inserted at an embryonic stage of said host animal.

The process of the invention may further comprise the steps of: mating said offspring with a normal conspecific nonhuman animal to obtain F1 transgenic animals, and mating said F1 transgenic animals among said conspecific F1 nonhuman animals.

In transgenic nonhuman animals of the invention, the thymus is greatly constricted and only residual materials or traces of structure similar to thymus remain. It is possible to obtain a transgenic nonhuman animal line in which mature T-cells are not discovered in the peripheral blood by technical manipulation as above. The transgenic nonhuman animal line is useful for research on diseases connected with the immune system such as AIDS as well as auto-immune diseases such as diabetes, immune inhibitors and provides a novel assay system therefor.

The following is presented by way of example, but does not limit the scope of the invention.
Figure 1 shows schematically a gene constructed to express human Hsp 70 under the regulation of an H2K promoter.
Figure 2 demonstrates the rough construction process for a human Hsp 70 gene expression vector;
Figure 3 demonstrates the analysis of tail DNA of a transgenic mouse by PCR; and
Figure 4 draws the flow cytometry analysis of lymphocytes isolated from spleen and blood of a transgenic mouse.

### Example 1: Construction of a human Hsp gene expression vector

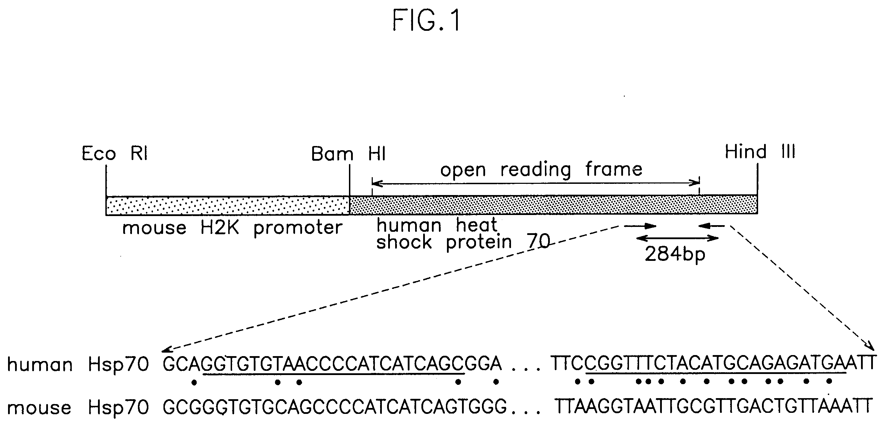

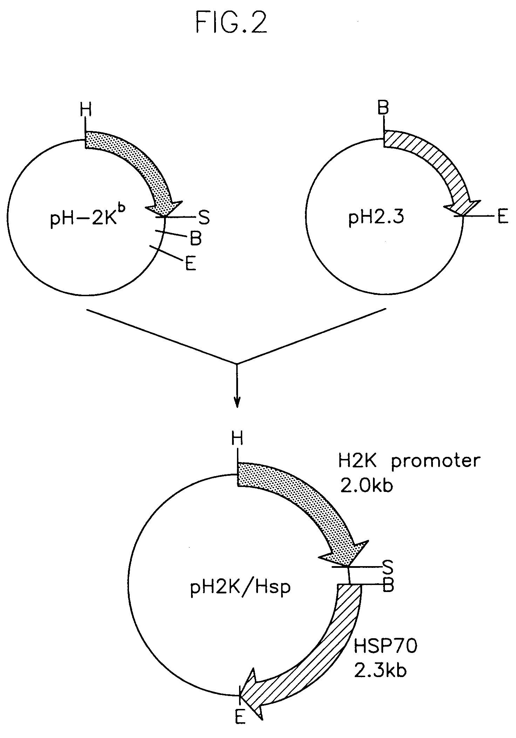

pH2.3 (Hunt, C. & Morimoto, R.I., 1985, Proc. Natl. Acad. Sci. 82 (19), 6455-6459) was digested with BamHI and HindIII restriction enzymes to obtain human Hsp gene, and a DNA fragment of 2.3 kb was obtained. pH2K (Morello, D. et al., 1986, EMBO. J. 5, 1877-1883) was treated with HindIII and EcoRI to obtain an H2K promoter of 2.0 kb. Two fragments were ligated to obtain a fragment of 4.3 kb (Figure 1). pH2K/HSP (Figure 2) was prepared by cloning the fragment of 4.3 kb into the restriction enzyme (EcoRI and HindIII) sites of pUC19 purchased from New England Biolabs, and after transformation in HB 101 E. coli, the transformed E. coli were stored. Ampicillin-containing LB medium (1% Bactotrypton, 0.5% Yeast Extract, 1% NaCl) was inoculated with the transformed E. coli to prepare a DNA solution for microinjection. The culture was incubated overnight at 37°C, at which time the culture was centrifuged for 10 minutes at 2000 g, collected, and dissolved in a solution of 50 mM glucose, 10 mM Tris-Cl, 1 mM EDTA, 4 mg/ml lysozyme and kept on ice for 5 minutes. This solution was carefully mixed with 2 volumes of 0.2 N NaOH containing 1% SDS solution and kept for 5 minutes at room temperature. To this, 0.5 volume of 5 M potassium acetate solution was added, and the mixture was kept on ice for 5 minutes. The supernatant was obtained by centrifugation at 12000 g for 10 minutes. The supernatant was extracted with phenol and phenol/chloroform/isoamylalcohol (25:24:1) twice or three times, and plasmid DNA was precipitated by adding 0.1 volume of 3M sodium acetate and 2 volumes of ethanol. The precipitated DNA was dissolved in TE buffer (10 mM Tris-Cl, pH 8.0/1 mM EDTA, pH 8.0) and CsCl was added to give 1 g/ml final concentration of CsCl. This solution was centrifuged at 100,000 rpm for 12 hours by a Beckman TL 100 rotor for purification of DNA. The band with plasmid DNA was separated with an injector, extracted with n-butanol and dialyzed for 24 hours against TE buffer. After plasmid DNA extraction, DNA was digested with the restriction enzyme EcoRI, purified by excising an agarose gel fragment containing H2K-Hsp 70 gene of 4.3 kb after electrophoresis on a 0.8% agarose gel. The concentration of DNA was quantified, DNA was diluted with 10 mM Tris-Cl, pH 7.5/0.2 mM EDTA, pH 8.0 buffer to become 4 ng/µl of DNA, stored in -20°C and used for injection.

### Example 2: Microinjection of H2K-Hsp gene

A great number of fertilized eggs were obtained from a few donor mice by superovulation of an F1 hybrid line (C57BL X CBA) purchased from Korean Life Engineering Research Institute or a female mouse of inbred FVB strain of at least 6 weeks postnatal age purchased from B&K Co. (England). The best time to microinject DNA into the male pronucleus of a fertilized egg cell is after 1 cell cycle, depending on the strain from the supplier and the light-dark cycle of the animal field. An F1 hybrid was used, the light-dark cycle of the animal room was set to be turned off at 7.00pm and turned on at 6.00am and microinjection was started at 10.30am. A fertilized egg was fixed at a position where a male pronucleus could be seen easily, and 1-2 pl DNA was injected into the male pronucleus with a pipet for injection.

### Example 3: Insertion of a microinjected fertilized egg into the oviduct of a foster mother mouse

Among the fertilized eggs with DNA inserted, dissolved eggs were discarded and the healthy eggs were transferred to a foster mother mouse on the same day or placed in M16 medium to culture at 37°C in an incubator. The line of male mouse to be mated with a foster mother mouse was an inbred ICR strain. A vasectomy was carried out on the male mouse, and the male mouse was used after ascertaining that a fetus was not produced despite the presence of a vaginal plug when it was mated with another female mouse. An ICR female mouse close to the ovulation period was mated with a male mouse after vasectomy, and if there was a vaginal plug, it was used as a foster mother mouse. The operation was carried out by injecting 0.2 ml of an anaesthetic, which is somnopentyl (64.8 mg sodium pentobarbital/ml produced by Pitman-Moore Co.) diluted 10 times with PBS, into the abdominal cavity, inserting 20 to 25 fertilized eggs, into which DNA had been microinjected, into both oviducts, and suturing the operated site.

### Example 4: Breeding of transgenic mice

A transgenic mouse was mated with a normal hybrid mouse (C57BL X CBA) or inbred FVB strain mouse which was at least 6 weeks postnatal in age after ascertaining through PCR of tail DNA that H2K-Hsp 70 gene was inserted. Every morning, the vaginal plug was examined for evidence of fertilization and, in case of an abnormal mating, it was examined again after being replaced with a normal mouse. Within 3-4 weeks after birth (F1), the transfer of H2K-Hsp 70 gene of a foster mother mouse was screened by PCR of tail DNA. After screening for the transfer of H2K-Hsp 70 gene, it was named the transgenic mice line. Again, an F1 transgenic mouse was mated with a normal hybrid (C57BL X CBA) mouse or inbred FVB strain at least 6 weeks postnatal in age.

Mating with the female pups of foster mother mice was carried out to prepare homozygotic transgenic mice. An F3 mouse was obtained by mating an F2 transgenic mouse born in the above and a normal hybrid (C57BL X CBA) mouse or inbred FVB strain mouse to search for homozygotic F2 transgenic mice, and to examine the percentage of mice with H2K-Hsp 70 gene.

### Example 5: Morphism analysis of a transgenic mouse deficient in T-cells

### (1) Tail DNA extraction

A DNA polymer was extracted from the tail of a mouse and the transformation gene was screened by PCR. 1.5-2.0 cm of the tail end of a mouse which was about 3-4 weeks old was excised, cut up finely, added to 500 µl buffer solution containing 50 mM of Tris-Cl, pH 8.0/50 mM of EDTA, and pH 8.0/0.5% of SDS. To this solution proteinase K was added to a concentration of 200 µg/ml, and the mixture was reacted at 55°C for 9 hours. The reactant was extracted with an equal volume of phenol and phenol/chloroform/isoamylalcohol (25:24:1) three times, and was dialysed against 10 mM of Tris-Cl, pH 8.0/1 mM of EDTA, pH 8.0 for 36 hours (12 hours X 3 times). The DNA concentration was measured by a spectrophotometer and this DNA solution was stored at 4°C.

### (2) PCR analysis

77.5 µl of deionized water, 2 µl of sample DNA, 2 µl each of dATP, dGTP, dCTP, dTTP, 10 µl of Taq DNA polymerase buffer (100 mM Tris (pH 8.3), 500 mM of KCl, 15 mM of MgCl₂, 0.1% gelatine), 2 µl of primer were mixed in a microfuge tube. The zone indicated by arrows in Figure 1 represents the primer and the underlined zone represents the base sequence of the primer. Mineral oil was added to the supernatant of the above solution and heat-treated at 95°C for 5 minutes to inactivate protease existing at DNA extraction. Subsequently, the temperature was adjusted to 72°C to denature DNA. 0.5 µl of Taq DNA polymerase (2.5 U) was added to the reaction mixture in each tube by passing through the oil layer, and a PCR cycle of denaturation (94°C, 0.5-1 minute), annealing (55°C, 0.5-1 minute) and extending (72°C, 1 minute) was carried out 30 times. After a pertinent PCR cycle was completed, the final step was prolonged for 5 minutes for an elongated DNA fragment to be induced and to form a double helix. Then the oil layer of the supernatant was removed, an equal volume of chloroform was added, and the mixture was centrifuged at 15,0000 rpm for 5 minutes. The supernatant was transferred to a new tube, 0.1 times of 3 M NaOAc (ph 5.2) and 2.5 times of 95% EtOH were added thereto, freezed at -70°C for 15 minutes and centrifuged at 15,000 rpm, at 4°C for 15 minutes. The precipitate was added to 70% EtOH, washed, centrifuged for 5 minutes and the final PCR elongated DNA was dried in a vacuum, dissolved in 20 µl of TE buffer or distilled water and 1-2 µl thereof was subjected to electrophoresis.

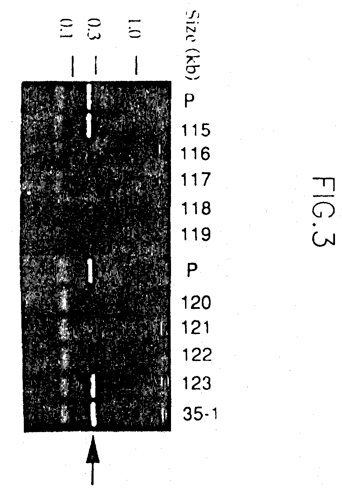

The experimental result is shown in Figure 3. P was a positive control group. H2K-Hsp 70 gene was successfully transferred to the above mice as the transgenic mice numbering 115, 123 and 35-1 showed the same bands as P.

### (3) Flow cytometry analysis of lymphocytes

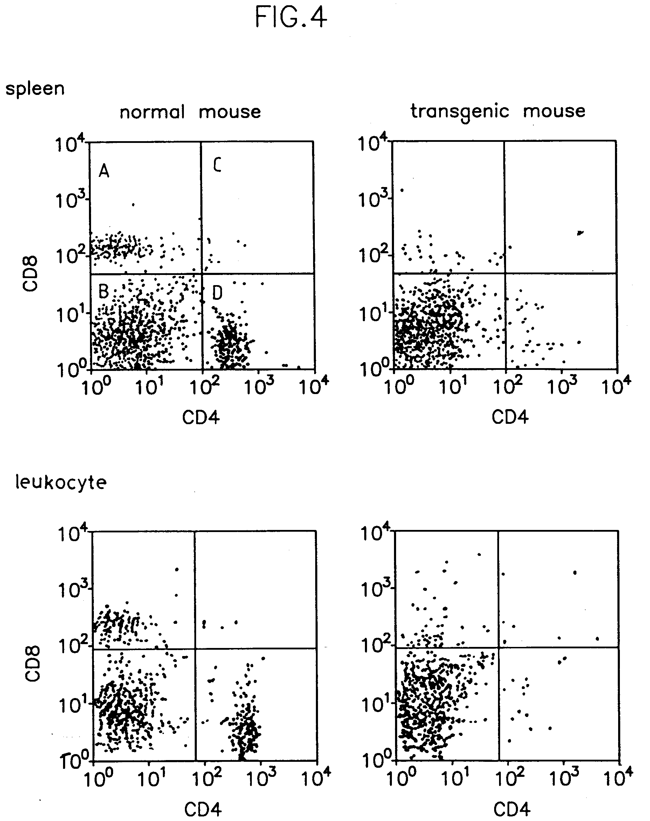

To examine whether a transgenic mouse is deficient in T-cells, lymphocytes were isolated from spleen and blood, where a lot of T-cells exist, and flow cytometry analysis was carried out using monoclonal antibodies specific to T cell markers CD-4 and CD-8 as shown in Figure 4.

The comparison between the results for a normal mouse (left) and a transgenic mouse (right), showed that the distributions differed. In particular, CD4 or CD8 recognition always exists among functional T-cells, as shown in the A region (lymphocytes having CD8, not CD4) or the D region (lymphocytes having CD4, not CD8). As the transgenic mouse did not show lymphocytes in the A region or the D region, it can be assumed that there were no functional T-cells in the transgenic mouse.

## Claims

1. A recombinant DNA comprising:
(a) a promoter of a gene expressed in thymus; and
(b) a heat shock protein 70 gene operatively linked downstream to said promoter.

2. The recombinant DNA of claim 1, wherein said promoter of a gene expresed in thymus is a promoter of an H2K gene.

3. The recombinant DNA of claim 2, wherein said H2K gene is a human H2K gene.

4. The recombinant DNA of any one of claims 1 to 3, wherein said heat shock protein 70 gene is a human heat shock protein 70 gene.

5. The recombinant DNA of any one of claims 1 to 4, wherein:
(a) said promoter is prepared by digesting an H2K gene with EcoRI and HindIII restriction enzymes;
(b) said heat shock protein 70 gene is prepared by digesting a heat shock protein 70 gene with BamHI and HindIII restriction enzymes; and
(c) the HindIII site of said H2K promoter is ligated to the HindIII site of said heat shock protein 70 gene.

6. A transgenic non-human animal comprising the recombinant DNA of any one of claims 1 to 5.

7. The transgenic non-human animal of claim 6 which is a mouse, a pig, or a chicken.

8. A process for preparing a transgenic non-human animal comprising the steps of:
(a) inserting the recombinant DNA of any one of claims 1 to 5 into a fertilized egg;
(b) transferring said fertilized egg to a foster mother; and
(c) obtaining the offspring of said foster mother.

9. The process of claim 8, wherein said recombinant DNA is inserted at an embryonic stage of said fertilized egg.

10. The process of claim 8 or 9, further comprising the steps of:
(a) mating said offspring with a normal conspecific non-human animal to obtain an F1 transgenic nonhuman animal; and
(b) mating said F1 transgenic non-human animal with another F1 transgenic non-human animal.

11. The process of any one of claims 8 to 10, wherein the transgenic non-human animal is a mouse, a pig or a chicken.

12. A transgenic non-human animal prepared by the process of any one of claims 8 to 11.
